# EUROPEAN PATENT SPECIFICATION

(11) **EP 1 024 823 B1**
(45) Date of publication and mention of the grant of the patent: **08.05.2002**
(21) Application number: 98951807.1
(22) Date of filing: 15.10.1998
(51) Int. Cl.: A61K 38/17, A61K 31/47, A61K 48/00, A61K 38/55, A61P 41/00

(54) **PREVENTION AND TREATMENT OF ADHESION FORMATION**
VORBEUGUNG UND BEHANDLUNG VON ADHÄSIONBILDUNG
PREVENTION ET TRAITEMENT DE L'ADHERENCE

(30) Priority: 16.10.1997 EP 97203217
(43) Date of publication of application: 09.08.2000
(73) Proprietor: NEDERLANDSE ORGANISATIE VOOR TOEGEPAST-NATUURWETENSCHAPPELIJK ONDERZOEK TNO, 2628 VK Delft (NL)
(72) Inventor: KOOISTRA, Teake, NL-2318 NH Leiden (NL)
(74) Representative: Prins, Adrianus Willem
(86) International application number: NL9800593
(87) International publication number: WO9920297

(56) References cited:
- US-A- 4 889 722
- US-A- 5 336 615
- US-A- 5 534 261
- SIMON D I ET AL: "Fibrin(ogen) is internalized and degraded by activated human monocytoid cells via Mac-1 (CD11b/CD18): A nonplasmin fibrinolytic pathway." BLOOD 82 (8). 1993. 2414-2422, XP002059464
- JACOBSEN L ET AL: "Molecular characterization of a novel human hybrid -type receptor that binds the alpha-2-macroglobulin receptor- associated protein." JOURNAL OF BIOLOGICAL CHEMISTRY 271 (49). 1996. 31379-31383, XP002059472

## Description

### Field of the invention

The present invention relates to the manufacture of medicaments for medical treatments to prevent or reduce the occurrence of adhesions to and between body organs, parts of body organs and/or tissues in mammals, in particular human beings, such as the adhesions occurring after surgical procedures.

### Background of the invention

The occurrence of intraperitoneal adhesions after abdominal surgery or peritoneal inflammation is a common cause of intestinal obstruction and impaired fertility in women, and can be a complicating factor in subsequent operations. Although not all etiological aspects are known, the sequence of events leading to intraperitoneal adhesions and peritoneal fibrosis is thought to start with fibrin deposition followed by immigration and proliferation of fibroblasts and collagen synthesis by fibroblasts. The fibrin deposits are the result of an imbalance between the procoagulant (fibrin-forming) and the fibrinolytic (fibrin-dissolving) activities of the peritoneum.

It has been reasoned that the restoration of the balance at an early stage of the disease process might lead to diminished fibrin deposition and thus might retard or prevent the fibroblast response and, consequently, adhesion formation.

To prevent adhesions of peritoneal surfaces, various pharmacological agents have been investigated or proposed, including quinacrine hydrochloride (U.S. Patent No. 5,478,837), corticosteroids, nonsteroidal anti-inflammatory drugs (Holz, Fertil. Steril. 37, 582, 1982), anticoagulants such as heparin (Jansen, Surg. Gynecol. Obstet. 166, 154, 1988), ibuprofen (U.S. Patent No. 4,346,108), antihistamines, antibiotics, dextran solutions, polyvinylpyrrolidone solutions and fibrinolytic agents (U.S. Patent No. 5,364,622).

With the exception of the fibrinolytic agents such as tissue-type plasminogen activator (t-PA), urokinase-type plasminogen activator (u-PA) and streptokinase, which appeared to be effective in animal models, results have so far been disappointing. These fibrinolytic agents convert the inactive zymogen plasminogen into plasmin. Plasmin effects the degradation of fibrin. Since fibrin deposition is a critical factor in the development of adhesions, administration of t-PA, u-PA or streptokinase results in the prevention of adhesion formation or removal of adhesions already formed.

This concept proved to be effective in the prevention of experimentally-induced peritoneal adhesions in animals, incl. rats (Evans et al., Am. J. Surg. 165, 229, 1993; Hill-West et al., J. Surg. Res. 59, 759, 1995), rabbits (Doody et al., Fertil. Steril. 51, 509, 1989; Menzies and Ellis, J. Royal. Soc. Med. 82, 534, 1989; Menzies and Ellis, Surg. Gynecol. Obstet. 172, 362, 1991; Dörr et al., Eur. J. Obstet. Gynecol. Reprod. Biol. 37, 287, 1990; Orita et al., Int. J. Fertil. 36, 172, 1991; Dunn and Mohler, J. Surg. Res. 54, 242, 1993) and dogs (Am. J. Obstet. Gynecol. 165, 1539, 1991).

Limitations and drawbacks of the administration of fibrinolysis activators are the relatively large amounts required, a transient time of action due to leakage from the cavity to plasma (Flessner et al., Am. J. Physiol. 240, H15, 1985) or inhibition by specific inhibitors, and, in the case of streptokinase, the evoked immune response.

An attractive alternative would be to increase the endogenous fibrinolytic capacity of a cavity in a regulated way during a desired period of time, but no effective strategies have been described up till now.

Mesothelial cells are the major cell population involved in the lining of the peritoneal cavity. It has been shown that cultured human mesothelial cells (HMC) produce t-PA and u-PA, and that the activity of these plasminogen activators in the medium is counteracted by the concomitant secretion of specific plasminogen activator inhibitors, including plasminogen activator inhibitor-1 (PAI-1).

We have discovered that HMC not only actively synthesize t-PA and u-PA, but also efficiently internalize and degrade t-PA and u-PA via receptor-mediated endocytosis. This is clearly different from the plasma situation, where t-PA is mainly or exclusively derived from one tissue, the vascular endothelium, but is cleared predominantly in another tissue, the liver.

Blockage of the internalization and degradation of t-PA and/or u-PA by HMC provides the possibility to increase the endogenous fibrinolytic activity.

The present invention provides the use of an active agent capable of providing for reduced internalization and degradation of plasminogen activators in mesothelial cells of a mammal, for preparing a pharmaceutical composition for reducing or preventing adhesions to or between organs, parts of organs or tissues, at a particular location, in said mammal.

### Summary of the invention

Preferably, said mammal is a human being, and said location is or has been exposed to a trauma which might lead to local fibrosis, such as a surgical procedure, e.g. abdominal surgery.

It is preferred that said active agent is capable of interfering with internalization of plasminogen activators by receptors on said mesothelial cells which receptors are involved in internalization of plasminogen activators, and/or interfering with recycling of said receptors, and/or interfering with degradation of plasminogen activators in said mesothelial cells. According to preferred embodiments, said active agent is selected from the group consisting of lysosomotropic agents such as chloroquine or a functional equivalent thereof, lysosomal proteinase inhibitors such as leupeptin or pepstatin, and other substances interfering with recycling or internalization of receptors, such as colchicine, cytochalasin B and monensin.

According to another preferred embodiment, said active agent is capable of blocking receptors on said mesothelial cells which receptors are involved in internalization of plasminogen activators. Herein, "blocking of receptors" means interfering with the binding between receptor and plasminogen activator. In a preferred embodiment, said active agent is Receptor Associated Protein (RAP) or a functional equivalent thereof. Said active agent may be a recombinant transfection or infection vector for said mesothelial cells, or other cells present at said location, which vector contains expressible nucleic acid encoding Receptor Associated Protein (RAP) or a functional equivalent thereof, to allow in vivo or ex vivo genetic modification of said cells and production of said RAP or functional equivalent thereof by said cells after their genetic modification. In an alternative, also preferred embodiment, said active agent is selected from the group consisting of an antibody, or a fragment thereof, binding to the plasminogen activator binding site of said receptors, and a peptide comprising or mimicking the receptor binding site of a plasminogen activator.

According to the present invention, the active agent may also be a plasminogen activator mutant which is resistant to receptor-mediated endocytosis in said mesothelial cells.

The invention further comprises that the active agent is a recombinant transfection or infection vector for said mesothelial cells, which vector contains nucleic acid encoding antisense mRNA for a mesothelial cell receptor involved in the internalization of plasminogen activators and allows in vivo or ex vivo genetic modification of said mesothelial cells such as to downregulate their expression of said receptor.

The invention also includes the use of an active agent capable of downregulating the expression of receptors on said mesothelial cells which receptors are involved in internalization of plasminogen activators.

### Brief description of the drawings

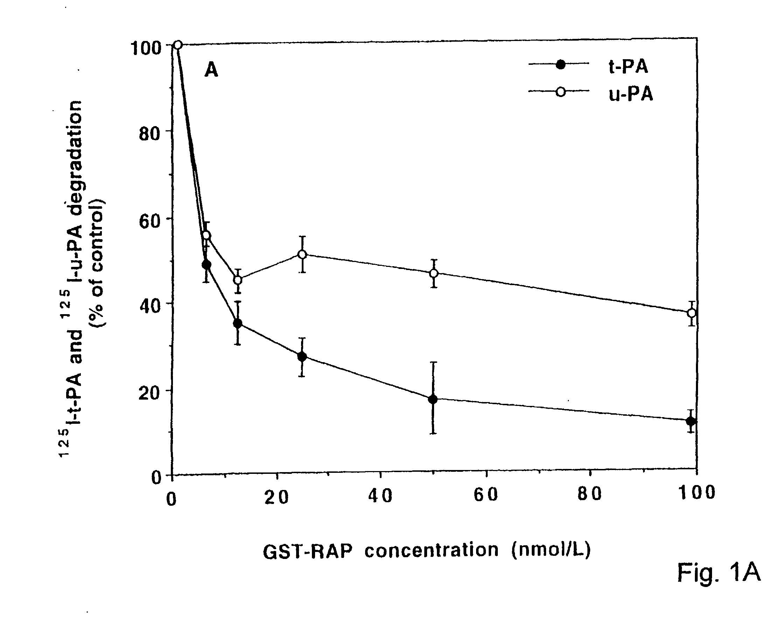

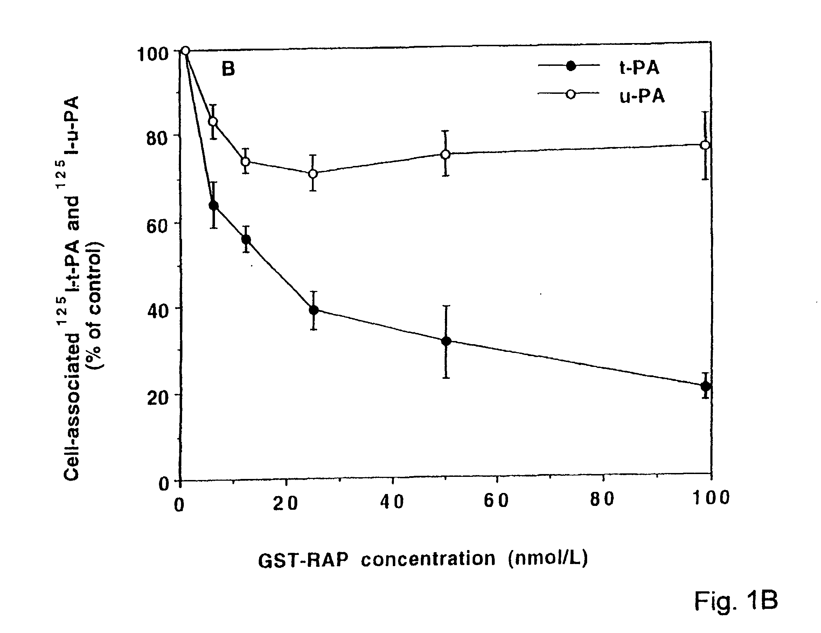

Fig. 1. HMC were incubated for 3 hours at 37°C with 2 nmol/L ¹²⁵I-t-PA or ¹²⁵I-u-PA, and different concentrations of GST-RAP. Inhibition of t-PA (•) and u-PA (o) degradation (A) and cell association (B) by GST-RAP was statistically significant (P ≤ .05) from 6.25 nmol/L onward. Data are expressed as percentage values of controls and are means ± SD of three independent experiments in triplicate.

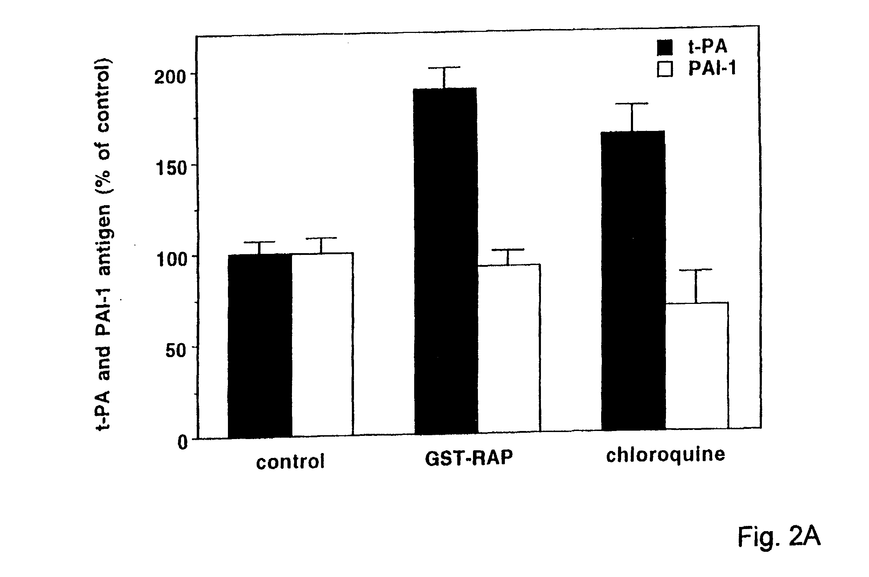

Fig. 2A. Effect of 100 nmol/L GST-RAP or 300 µmol/L chloroquine on the accumulation of t-PA and PAI-1 in the conditioned media of HMC. HMC were incubated for 24 hours with or without GST-RAP or chloroquine, and the conditioned media assayed for the presence of t-PA antigen (■) and PAI-1 (□). Data are expressed as percentage values of controls and are means ± SD of three independent experiments in duplicate.

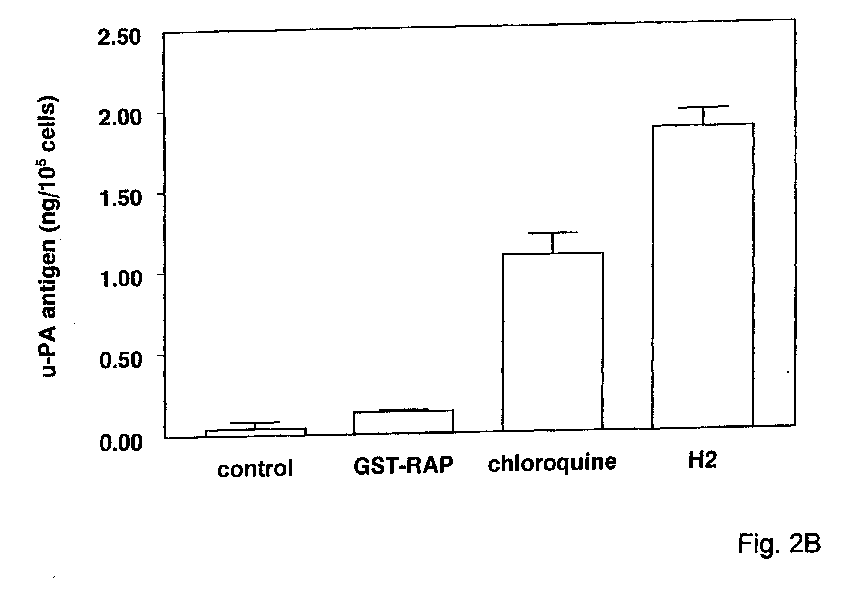

Fig. 2B. Effect of 1 µmol/L GST-RAP, 300 µmol/L chloroquine and 5 µg/mL u-PA receptor blocking antibody H-2 on the accumulation of u-PA in the conditioned media of HMC. HMC were incubated for 24 hours with GST-RAP, chloroquine or H-2, and the conditioned media assayed for the presence of u-PA antigen. The data shown are from one representative experiment of three performed and are expressed as means ± SD of a triplicate experiment.

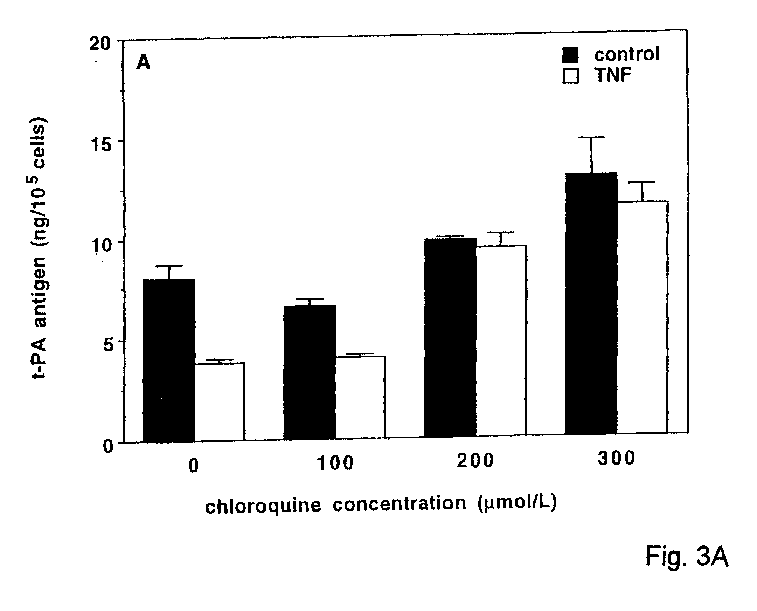

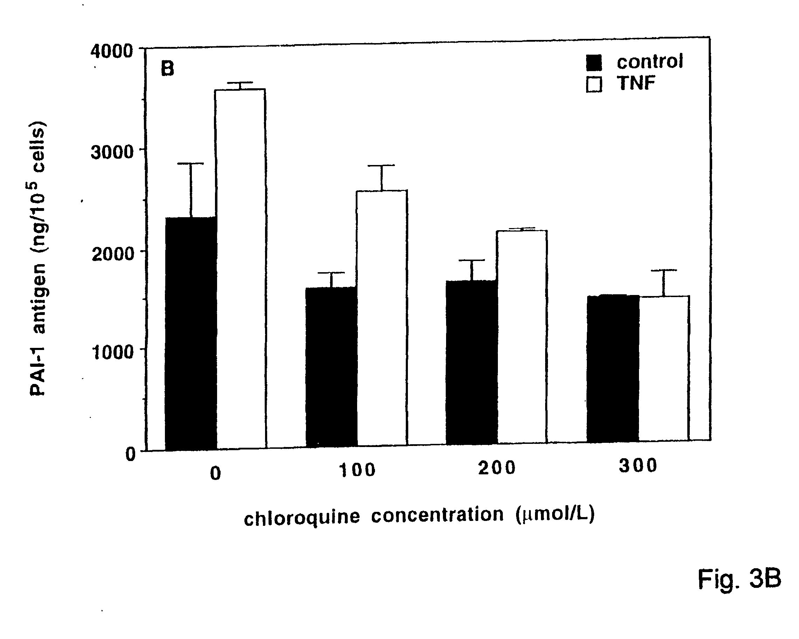

Fig. 3. HMC were incubated for 24 hours at 37°C with or without 1000 U/mL TNFα and increasing concentrations of chloroquine (100-300 µmol/L), which was added 30 minutes prior to TNFα. The conditioned media were analyzed for t-PA antigen (A) and PAI-1 antigen (B). The data shown are from one representative experiment of three performed and are expressed as mean values of a duplicate experiment, with ranges indicated by error bars.

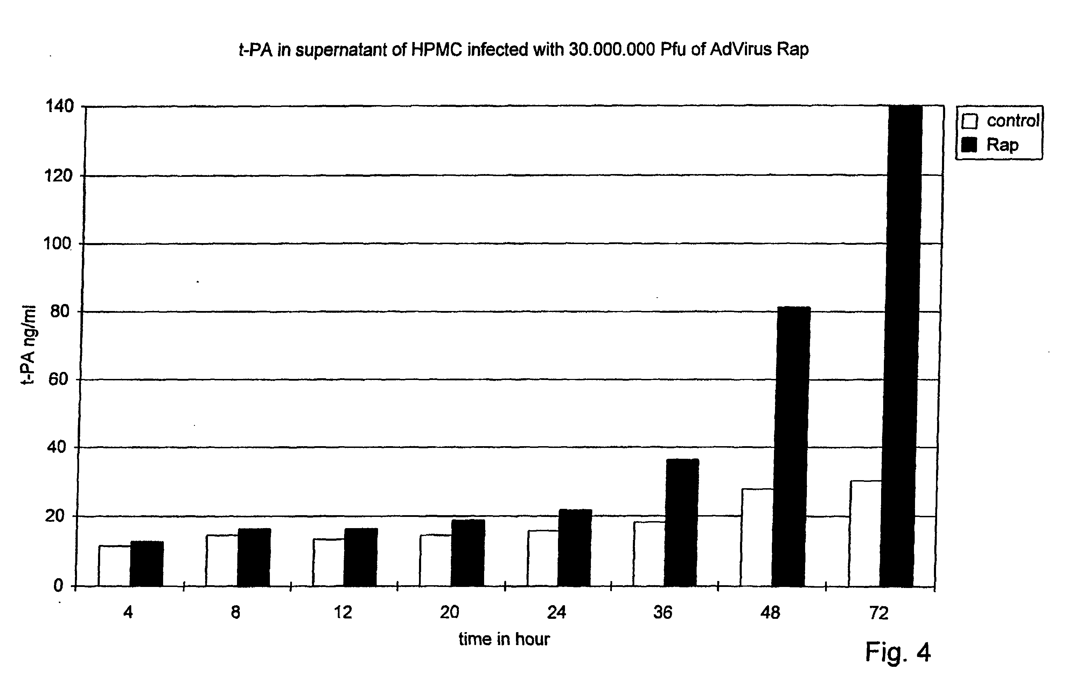

Fig. 4. HMC (2 cm²) were incubated with 200 µl M199 medium/2% (vol/vol) new-born calf serum containing 3x10⁷ plaque forming units (pfu) per ml of AdCMV-RAP. After 1 hour, cells were washed twice with M199 and the cells refed with culture medium. HMC were incubated for the indicated times, and the conditioned media of control (non-infected) cells and infected cells assayed for t-PA antigen. The data shown are from one representative experiment of three performed and are expressed as mean values of a triplicate experiment.

### Detailed description of the invention

As a result of extensive studies we established the following findings.

Internalization and degradation of t-PA and u-PA in HMC is strongly inhibited in the presence of lysosomotropic agents, such as in particular chloroquine.

Addition of recombinant 39-kD receptor-associated protein (RAP, inhibitor of low-density-lipoprotein receptor-related protein [LRP] and of very low-density-lipoprotein [VLDL] receptor) almost completely blocks the degradation of t-PA and that of u-PA.

Incubation of HMC in the presence of chloroquine or RAP increases the levels of t-PA and u-PA in conditioned medium.

The inflammatory mediator tumor necrosis factor α (TNFα) diminishes the fibrinolytic capacity of HMC by decreasing the synthesis of t-PA and increasing that of PAI-1. This fall in t-PA levels can be counteracted by inhibiting t-PA (and u-PA) degradation by either chloroquine or RAP. In addition, chloroquine quenches the TNFα-induced decrease in t-PA and increase in PAI-1 expression (at the level of antigen and mRNA).

Infection with RAP-adenovirus also results in enhanced levels of t-PA (and u-PA) in the conditioned medium of HMC.

The invention proposes a new concept to increase the endogenous fibrinolytic capacity of body cavities by blocking the degradation of fibrinolysis activators and is applicable in the prevention of adhesion formation.

The invention can be applied in various ways to prevent adhesions of (sections of) tissue and/or organs in various situations. Briefly,

In the case of surgery:
- An inhibitor of receptor-mediated endocytosis of t-PA and/or u-PA, for example chloroquine or RAP, may be applied topically in the area of surgical intervention during surgery or immediately after surgery has ended.
- The cells lining the peritoneal cavity, including mesothelial cells, may be infected with RAP-adenovirus (or other transfection or infection systems) during or immediately after surgery.
- Cells, for example mesothelial cells, may be infected as above, but ex vivo. The genetically modified cells are then reseeded on the peritoneal surface during surgery or after surgery has ended.
- The expression of receptors involved in the uptake and degradation of t-PA and/or u-PA may be downregulated, for example pharmacologically or by antisense technology (before, during or after surgery).
- Mutants of t-PA and/or u-PA that are resistant to receptor-mediated endocytosis may be applied topically in the area of surgical intervention during surgery or immediately after surgery has ended.

Other cases, for example inflammatory processes during CAPD:
- A compound or composition according to the invention may be applied through a catheter which ends at the site of intervention.

Cavities other than the peritoneal cavity:
- The application of the invention is not restricted to the peritoneum but is intended to include any relevant part of a living animal including but not limited to lung and heart.

The compositions and methods according to the present invention are useful in reducing, minimizing or preventing adhesion formation, the most common cause of which is prior surgery. The compositions and methods according to the present invention have been shown to be especially effective in preventing the formation of adhesions between organ surfaces, in particular adhesion formation in the peritoneum following surgery. The present invention also finds utility in other contexts, however, such as e.g. for cardiovascular, orthopedic, thoracic, ophthalmic, central nervous system and other uses where prevention of the formation of adhesions is a significant concern. In the following discussion, attention is directed primarily to a description of compositions and methods useful in inhibiting peritoneal adhesion formation, but the various possibilities and preferred embodiments disclosed therein apply to all other conditions where the invention is applicable.

The term "pharmaceutical composition" as used herein is intended to cover both the active agent alone and a composition containing the active agent together with a pharmaceutically acceptable carrier, diluent or excipient.

Pursuant to one embodiment of the present invention, chloroquine is administable in an effective concentration at the site of potential adhesion formation. The administration may be done as a single dose, as a discontinuous sequence of various doses, or continuously for a period of time sufficient to permit substantial tissue repair, in particular re-epithelialization or mesothelial repair. The active agent is typically administable during the surgical procedure up to some time after completion thereof, and administration may even be carried out or initiated shortly before surgery. In the case of a continuous administration, the duration of the administration may vary depending upon a number of factors which would readily be appreciated by those skilled in the art. In general, the administration of a composition in accordance with the present invention should be effected from the time of surgery for at least one or two days after completion of the surgical procedure. As healing is in most cases complete within about two weeks, it is generally not necessary to continue administration of a composition in accordance with the present invention much longer than two weeks. Preferably, a composition according to the present invention is administrable from about the time of surgery for a period of about one day to about two weeks, such as more particularly for a period of about two days to about one week.

The administration dose of the active agent may be varied over a fairly broad range. The concentrations of chloroquine which can be administered would be limited by efficacy at the lower end and the solubility of the compound at the upper end. The optimal dose or doses for a particular patient should and can be determined by the physician or medical specialist involved, taking into consideration well known relevant factors such as the condition, weight and age of the patiënt, the given or predicted extent of the adhesions, etc.

The active agent may be administered directly in a suitable vehicle, such as e.g. phosphate-buffered saline (PBS). Pursuant to preferred embodiments of the present invention, however, the active agent is administrable through a single dose delivery using a drug-delivery system which enables the maintenance of the required concentrations of the active agent for a period of time sufficient for adequate tissue repair. A suitable drug-delivery system would be pharmacologically inactive or at least tolerable. It should preferably not be immunogenic nor cause inflammatory reactions, and should permit release of chloroquine so as to maintain effective levels thereof over the desired time period. A large variety of alternatives are known in the art as suitable for purposes of sustained release and are comtemplated as within the scope of the present invention. Suitable delivery vehicles include, but are not limited to, the following: microcapsules or microspheres; liposomes and other lipid-based release systems; viscous instillates; absorbable and/or biodegradable mechanical barriers and implants; and polymeric delivery materials, such as polyethylene oxide/polypropylene oxide block copolymers, polyesters, crosslinked polyvinylalcohols, polyanhydrides, polymethacrylate and polymethacrylamide hydrogels, anionic carbohydrate polymers, etc. Useful delivery systems are well known in the art.

A highly suitable formulation to achieve the active agent release comprises injectable microcapsules or microspheres made from a biodegradable polymer, such as poly(dl-lactide), poly(dl-lactide-co-glycolide), polycaprolactone, polyglycolide, polylactic acid-co-glycolide, poly(hydroxybutyric acid), polyesters or polyacetals. Injectable systems comprising microcapsules or microspheres having a diameter of about 50 to about 500 micrometers offer advantages over other delivery systems. For example, they generally use less active agent and may be administered by paramedical personnel. Moreover, such systems are inherently flexible in the design of the duration and rate of separate drug release by selection of microcapsule or microsphere size, drug loading and dosage administered. Further, they can be successfully sterilized by gamma irradiation.

The design, preparation and use of microcapsules and microspheres are well within the reach of persons skilled in the art and detailed information concerning these points is available in the literature.

Biodegradable polymers (such as lactide, glycolide and caprolactone polymers) may also be used in formulations other than microcapsules and microspheres; e.g. pre-made films and spray-on films of these polymers containing the active agent would be suitable for use in accordance with the present invention. Fibers or filaments comprising the active agent are also comtemplated as within the scope of the present invention.

Another highly suitable formulation for a single-dose delivery of the active agent in accordance with the present invention involves liposomes. The encapsulation of an active agent in liposomes or multilamellar vesicles is a well known technique for targeted drug delivery and prolonged drug residence. The preparation and use of drug-loaded liposomes is well within the reach of persons skilled in the art and well documented in the literature.

Yet another suitable approach for single dose delivery of chloroquine in accordance with the present invention involves the use of viscous instillates. In this technique, high molecular weight carriers are used in admixture with the active agent, giving rise to a structure which produces a solution with high viscosity. Suitable high molecular weight carriers include, but are not limited to, the following: dextrans and cyclodextrans; hydrogels; (cross-linked) viscous materials, including (cross-linked) viscoelastics; carboxymethylcellulose; hyaluronic acid; and chondroitin sulfate. The preparation and use of drug-loaded viscous instillates is well known to persons skilled in the art.

Pursuant to yet another approach, the active agent may be administered in combination with absorbable mechanical barriers such as oxidized regenerated cellulose. The active agent may be covalently or non-covalently (e.g. ionically) bound to such a barrier, or it may simply be dispersed therein.

The above described approaches for administration of chloroquine apply in general to any active agent in accordance with the present invention. It applies to any lysosomotropic agent or, more generally, any substance capable of interfering with internalization of plasminogen activators through their receptors on mesothelial cells, or capable of interfering with the recycling of these receptors after their internalization, or both. For example, it applies to compounds like colchicine, cytochalasin B and monensin, and to inhibitors of lysosomal proreinases such as leupeptin and pepstatin. It further applies to (recombinant) RAP and other substances capable of blocking the receptors on mesothelial cells involved in plasminogen activator internalization, including antibodies (or fragments thereof) capable of binding to the plasminogen activator recognition site on these receptors, peptides comprising or mimicking the receptor binding site of plasminogen activators, and more generally all compounds that prevent interaction and may be found by screening procedures. It applies to any other agent capable of reducing or inhibiting internalization and degradation of t-PA and/or u-PA by HMC, and to active agents capable of downregulating in HMC the expression of receptors involved in the uptake and degradation of t-PA and/or u-PA, and to mutants of t-PA and u-PA that are resistant to receptor-mediated endocytosis in HMC.

The 39-kD receptor-associated protein RAP may alternatively be administered by using gene therapy technology. In accordance therewith, a suitable transfection or infection system is used to introduce expressible RAP-encoding nucleic acid into the mesothelial cells of the patient to be treated. Suitable transfection or infection systems are well known to the person skilled in the art and comprise, e.g. adenovirus. RAP-encoding nucleic acid is available in the art. The construction of a recombinant adenovirus containing RAP-encoding nucleic acid, which recombinant virus would allow the expression of RAP in transfected HMC, is described by J. Herz, Science 264, 1471, 1994.

The transfection or infection process may be carried out in situ, with the HMC in their natural place, or ex vivo with HMC removed from the body of the patient and reseeded after their genetic modification.

Modern techniques of genetic engineering may also be used to downregulate the expression of receptors on HMC that are involved in the internalization and degradation of plasminogen activators. A well known technique, which would be suitable for this purpose, is the so-called antisense technique. Pursuant to this technique, the HMC cells of interest should be genetically modified so as to produce antisense messengers capable of blocking a gene or mRNA responsible for the expression of said receptors.

The examples provided herein are merely intended to be illustrative and should not be viewed as in any sense limiting the scope of the invention, which is defined hereinafter in the accompanying claims.

### Examples

Multiple studies were performed to demonstrate that blocking of receptor-mediated endocytosis of t-PA and/or u-PA leads to increases in the levels of t-PA and/or u-PA in the conditioned medium of human mesothelial cells (HMC). The model system used consisted of cultured human peritoneal mesothelial cells which were isolated from the omental tissue of consenting patients undergoing elective surgery. Cells were grown in fibronectin-coated dishes in M199 supplemented with 20 mmol/L HEPES (pH 7.4), 10% (vol/vol) human serum, 10% (vol/vol) heat-inactivated newborn calf serum, 150 µg/mL endothelial cell growth supplement, 2 mmol/L L-glutamine, 5 IU/mL heparin, 100 U/mL penicillin, and 100 µg/mL streptomycin at 37°C in a 5% CO₂/95% air atmosphere. The medium was replaced every 2 to 3 days. Subcultures were obtained by trypsin/EDTA treatment of confluent monolayers at a split ratio of 1:3. Cells from omental tissue were pure mesothelial cells as assessed by their uniform cobblestone appearance at confluence, by the absence of von Willebrand factor, and the uniform positive staining for cytokeratins 8 and 18 and for vimentin (Van Hinsbergh et al., Blood 75, 1490, 1990). For the experiments, confluent cultures were used at the second or third passage, and cells were always re-fed the day before the experiment with incubation medium: M199, supplemented with 20 mmol/L HEPES (pH 7.4), 10% (vol/vol) human serum, 2 mmol/L L-glutamine, and antibiotics. Conditioned media were obtained by incubating cells in 2 cm² dishes at 37°C with 0.5 mL incubation medium containing the appropriate concentration of the test compound or stock solvent. The conditioned media were centrifuged for 5 minutes at 8,000 rpm in a Microfuge centrifuge to remove cells and cellular debris. Conditioned media were stored at -20°C until use.

### Example 1

One way to circumvent the rapid uptake of t-PA and u-PA by mesothelial cells is to administer a specific inhibitor of LRP, for example receptor-associated protein (RAP) (Moestrup and Gliemann, J. Biol. Chem. 266, 21232, 1991; Williams et al., J. Biol. Chem. 267, 9035, 1992; Warshawsky et al., J. Clin. Invest. 92, 937, 1993). We measured the degradation and cell-association of radiolabelled t-PA and radiolabelled u-PA in the presence of glutathione-S-transferase (GST)-RAP and D-mannose. GST-RAP was prepared at our laboratory using the Salmonella japonicum glutathione-S-transferase (GST)-RAP expression plasmid, kindly provided by Dr J.Herz (University of Texas Southwestern Medical Center, Dallas, TX) (Herz et al., J. Biol. Chem. 266, 21232, 1991).

The results shown in Figure 1A demonstrate that addition of GST-RAP to the incubated cells inhibited in a concentration-dependent manner the degradation of ¹²⁵I-t-PA and ¹²⁵I-u-PA. At the highest concentration (100 nmol/L) the competitive effect of GST-RAP on the degradation of ¹²⁵I-t-PA was as strong as the addition of excess unlabeled t-PA (89% vs 91% inhibition). Less effect of GST-RAP was observed on the degradation of ¹²⁵I-u-PA, where degradation was reduced maximally to 35%. Parallel measurement of the amount of cell-associated radiolabelled t-PA and u-PA showed a very comparable picture (Fig. 1B). At the highest concentration of GST-RAP (100 nmol/L), the reduction of cell-associated ¹²⁵I-t-PA was similar to that seen in the presence of an excess (200 nmol/L) of unlabelled t-PA (84%). In agreement with the degradation studies, GST-RAP was less effective in lowering ¹²⁵I-u-PA association to HMC (reduction to 76%), while unlabeled u-PA (200 nmol/L) reduced u-PA binding to 4%. This discrepancy suggests the presence of an additional u-PA clearing system in HMC, which could obviously not be blocked by GST-RAP. Even at a concentration of 100 nmol/L, D-mannnose could not block the degradation of either t-PA or u-PA (data not shown), confirming the absence of significant mannose receptor expression in HMC. Parallel experiments with liver endothelial cells have shown that this concentration of D-mannose is sufficient to prevent degradation of t-PA.

### Example 2

We measured t-PA antigen accumulation in the conditioned media of HMC after 24 h incubation in the presence of GST-RAP (100 nmol/L) or chloroquine (300 µmol/L) (Fig 2A). As a result of the reduced degradation of t-PA, antigen levels were nearly two-fold higher in the media of cells incubated with GST-RAP or chloroquine relative to those of untreated control cultures. PAI-1 antigen levels did not alter significantly in the presence of GST-RAP and were only slightly decreased with chloroquine, indicating that the increases in t-PA levels were not the result of a general enhancement of protein synthesis.

Addition of either chloroquine (300 µmol/L) or the u-PA receptor blocking antibody H-2 (5 µg/mL) increased u-PA levels in a representative experiment from 0.04 ± 0.04 ng/10⁵ cells in controls to 1.08 ± 0.13 and 1.86 ± 0.11 ng/10⁵ cells in the presence of chloroquine and H-2, respectively (Fig. 2B). These findings confirm the critical role of u-PA receptor in u-PA degradation, but also illustrate the potent u-PA degradative capacity of HMC. GST-RAP., which increased t-PA accumulation as effectively as chloroquine (see also Fig. 2A), prevented u-PA, degradation to a much lesser extent, thus underlining the existence of an additional, u-PA receptor-dependent u-PA clearing system in HMC.

### Example 3

We have previously shown that TNFα reduces fibrinolytic activity of HMC by increasing PAI-1 and decreasing t-PA synthesis (Sitter et al., Am. J. Physiol. 271, R1256, 1996). To check whether inhibition of degradation by GST-RAP or chloroquine could overcome the TNFα-induced decrease in t-PA synthesis in HMC, we found that, apart from inhibiting t-PA degradation, chloroquine also quenches the action of TNFα. When added simultaneously with TNFα (1000 U/ml), chloroquine inhibited in a concentration-dependent manner (100-300 µmol/L) both the TNFα-evoked decrease in t-PA production and the TNFα-induced increase in PAI-1 production (Fig 3). No such effect was seen with GST-RAP which only counteracted the TNFα-induced decrease in t-PA antigen by inhibiting degradation (data not shown).

### Example 4

We have used recombinant adenovirus expressing the full length rat RAP cDNA (AdCMV-RAP) (provided by Dr. J. Herz, Science 264, 1471, 1994) to carry a RAP cDNA into cultured human mesothelial cells to inactivate LRP and cause t-PA (and u-PA) to accumulate in the medium. Recombinant adenovirus have proven to be efficient gene transfer vectors.

Infection of human mesothelial cells was performed in 24 well dishes (2 cm²). Cells were incubated with 200 µl M199 medium/2% (vol/vol) new-born calf serum containing AdCMV-RAP in concentrations ranging between 1x10⁶ and 1x10⁹ plaque forming units (pfu) per ml. After 1 hour, cells were washed twice with M199 and the cells re-fed with culture medium, viz. M199 supplemented with 20 mmol/L HEPES (pH 7.4), 10% (vol/vol) human serum, 10% (vol/vol) heat-inactivated new-born calf serum, 150 µg/mL endothelial cell growth supplement, 2 mmol/L L-glutamine, 5 IU/mL heparin, 100 U/mL of penicillin and 100 µg/mL of streptomycin.

Cells were kept at 37°C in a 5% CO₂/95% air atmosphere. Conditioned medium was collected every 24 hours and replaced by fresh culture medium. As shown in Fig. 4, t-PA accumulation in the conditioned medium of Ad CMV-RAP-infected cells was clearly increased as compared to control cells.

## Claims

1. Use of an active agent capable of providing for reduced internalization and degradation of plasminogen activators in mesothelial cells of a mammal, for preparing a pharmaceutical composition for reducing or preventing adhesions to or between organs, parts of organs or tissues, at a particular location, in said mammal.

2. Use according to claim 1, wherein said mammal is a human being.

3. Use according to claim 1, wherein said location is or has been exposed to a trauma, e.g. a surgical procedure, which might lead to local fibrosis.

4. Use according to claim 1, wherein said active agent is capable of interfering with internalization of plasminogen activators by receptors on said mesothelial cells which receptors are involved in internalization of plasminogen activators, and/or interfering with recycling of said receptors, and/or interfering with degradation of plasminogen activators in said mesothelial cells.

5. Use according to claim 4, wherein said active agent is selected from the group consisting of lysosomotropic agents such as chloroquine or a functional equivalent thereof, lysosomal proteinase inhibitors such as leupeptin or pepstatin, and other substances interfering with recycling or internalization of receptors, such as colchicine, cytochalasin B and monensin.

6. Use according to claim 1, wherein said active agent is capable of blocking receptors on said mesothelial cells which receptors are involved in internalization of plasminogen activators.

7. Use according to claim 6, wherein said active agent is Receptor Associated Protein (RAP) or a functional equivalent thereof.

8. Use according to claim 6, wherein said active agent is a recombinant transfection or infection vector for said mesothelial cells, or other cells present at said location, which vector contains expressible nucleic acid encoding Receptor Associated Protein (RAP) or a functional equivalent thereof, to allow in vivo or ex vivo genetic modification of said cells and production of said RAP or functional equivalent thereof by said cells after their genetic modification.

9. Use according to claim 6, wherein said active agent is selected from the group consisting of an antibody, or a fragment thereof, binding to the plasminogen activator binding site of said receptors, and a peptide comprising or mimicking the receptor binding site of a plasminogen activator.

10. Use according to claim 1, wherein said active agent is a plasminogen activator mutant which is resistant to receptor-mediated endocytosis in said mesothelial cells.

11. Use according to claim 1, wherein said active agent is a recombinant transfection or infection vector for said mesothelial cells, which vector contains nucleic acid encoding antisense mRNA for a mesothelial cell receptor involved in the internalization of plasminogen activators and allows in vivo or ex vivo genetic modification of said mesothelial cells such as to downregulate their expression of said receptor.

12. Use according to claim 1, wherein said active agent is capable of downregulating the expression of receptors on said mesothelial cells which receptors are involved in internalization of plasminogen activators.

## Patentansprüche

1. Verwendung eines Wirkstoffs, der in der Lage ist, eine reduzierte Internalisierung und Degradation von Plasminogenaktivatoren in mesothelialen Zellen eines Säugers zu bewirken, zur Herstellung einer pharmazeutischen Zusammensetzung zur Reduktion oder Verhinderung von Adhäsionen an oder zwischen Organen, Teilen von Organen oder Geweben in einer bestimmten Lokalisierung in diesem Säuger.

2. Verwendung nach Anspruch 1, bei der der Säuger ein Mensch ist.

3. Verwendung nach Anspruch 1, bei der die Lokalisierung einem Trauma ausgesetzt ist oder war, z.B. einem chirurgischen Eingriff, der zu einer lokalen Fibrose führen kann.

4. Verwendung nach Anspruch 1, bei der der Wirkstoff in der Lage ist, mit der Internalisierung von Plasminogenaktivatoren durch Rezeptoren auf den mesothelialen Zellen, deren Rezeptoren an der Internalisierung von Plasminogenaktivatoren beteiligt sind, zu interferieren, und/oder mit dem Recycling der Rezeptoren zu interferieren, und/oder mit der Degradation der Plasminogenaktivatoren in den mesothelialen Zellen zu interferieren.

5. Verwendung nach Anspruch 4, bei der der Wirkstoff aus der Gruppe bestehend aus lysosomotropen Agenzien, wie beispielsweise Chloroquin, oder einem funktionellen Äquivalenten davon, lysosomalen Proteinase-Inhibitoren, wie beispielsweise Leupeptin oder Pepstatin, und anderen Substanzen, die mit dem Recycling oder der Internalisierung der Rezeptoren interferieren, wie beispielsweise Colchicin, Cytochalasin B und Monensin.

6. Verwendung nach Anspruch 1, bei der der der Wirkstoff in der Lage ist, Rezeptoren auf den mesothelialen Zellen, deren Rezeptoren an der Internalisierung der Plasminogenaktivatoren beteiligt sind, zu blockieren.

7. Verwendung nach Anspruch 6, bei der der Wirkstoff das Rezeptor-assoziierte Protein (RAP) oder ein funktionelles Äquivalent davon ist.

8. Verwendung nach Anspruch 6, bei der der Wirkstoff ein rekombinanter Transfektions- oder Infektionsvektor für die mesothelialen Zellen oder andere Zellen ist, die an der besagten Lokalisierung vorhanden sind, wobei der Vektor eine exprimierbare Nukleinsäure enthält, die für Rezeptor-assoziiertes Protein (RAP) oder ein funktionelles Äquivalent desselben kodiert, um in vivo oder ex vivo die genetische Modifikation der Zellen und die Produktion des RAP oder funktionellen Äquivalents desselben durch die Zellen nach deren genetischer Modifikation zu erlauben.

9. Verwendung nach Anspruch 6, bei der der Wirkstoff aus der Gruppe bestehend aus einem Antikörper oder einem Fragment desselben, die an die Plasminogenaktivator-Bindungsstelle der Rezeptoren binden, und einem Peptid, das die Rezeptorbindungsstelle eines Plasminogenaktivators umfasst oder nachahmt.

10. Verwendung nach Anspruch 1, bei der der Wirkstoff eine Plasminogenaktivator-Mutante ist, die gegen Rezeptor-vermittelte Endozytose in den mesothelialen Zellen resistent ist.

11. Verwendung nach Anspruch 1, bei der der Wirkstoff ein rekombinanter Transfektions- oder Infektionsvektor für die mesothelialen Zellen ist, wobei der Vektor Nukleinsäure enthält, die für Antisense mRNA für einen an der Internalisierung von Plasminogenaktivatoren beteiligten Mesothelzell-Rezeptor kodiert, und in vivo oder ex vivo die genetische Modifikation der besagten mesothelialen Zellen ermöglicht, um beispielsweise ihre Expression des Rezeptors herabzuregulieren.

12. Verwendung nach Anspruch 1, **dadurch gekennzeichnet, dass** der Wirkstoff in der Lage ist, die Expression von Rezeptoren auf den mesothelialen Zellen, deren Rezeptoren an der Internalisierung von Plasminogenaktivatoren beteiligt sind, herabzuregulieren.

## Revendications

1. Utilisation d'une substance active capable d'assurer une internalisation et une dégradation réduites des activateurs du plasminogène dans les cellules mésothéliales d'un mammifère, pour la préparation d'une composition pharmaceutique destinée à réduire ou prévenir les adhérences à ou entre organes, parties d'organes ou tissus, à un site particulier, chez ledit mammifère.

2. Utilisation selon la revendication 1, dans laquelle ledit mammifère est un être humain.

3. Utilisation selon la revendication 1, dans laquelle ledit site est ou a été exposé à un traumatisme, par exemple une intervention chirurgicale, susceptible de conduire à une fibrose locale.

4. Utilisation selon la revendication 1, dans laquelle ladite substance active est capable de perturber l'internalisation des activateurs du plasminogène par les récepteurs présents sur lesdites cellules mésothéliales, lesquels récepteurs sont impliqués dans l'internalisation des activateurs du plasminogène, et/ou de perturber le recyclage desdits récepteurs et/ou de perturber la dégradation des activateurs du plasminogène dans lesdites cellules mésothéliales.

5. Utilisation selon la revendication 4, dans laquelle ladite substance active est choisie dans le groupe comprenant des agents lysosomotropes tels que la chloroquine ou un équivalent fonctionnel de ceux-ci, des inhibiteurs de la protéinase lysosomale tels que la leupeptine ou la pepstatine, et d'autres substances perturbant le recyclage ou l'internalisation des récepteurs telles que la colchicine, la cytochalasine B et la monensine.

6. Utilisation selon la revendication 1, dans laquelle ladite substance active est capable de bloquer les récepteurs présents sur lesdites cellules mésothéliales, lesquels récepteurs sont impliqués dans l'internalisation des activateurs du plasminogène.

7. Utilisation selon la revendication 6, dans laquelle ladite substance active est la protéine associée aux récepteurs (PAR) ou un équivalent fonctionnel de celle-ci.

8. Utilisation selon la revendication 6, dans laquelle ladite substance active est un vecteur de transfection ou d'infection recombinant pour lesdites cellules mésothéliales, ou d'autres cellules présentes au niveau de ce site, lequel vecteur contient un acide nucléique exprimable codant pour la protéine associée aux récepteurs (PAR) ou un équivalent fonctionnel de celle-ci, afin de permettre la modification génétique in vivo ou ex vivo desdites cellules et la production de ladite PAR ou dudit équivalent fonctionnel de celle-ci par lesdites cellules après leur modification génétique.

9. Utilisation selon la revendication 6, dans laquelle ladite substance active est choisie dans le groupe comprenant un anticorps ou un fragment de celui-ci qui se lie au site de liaison des activateurs du plasminogène desdits récepteurs, et un peptide comprenant ou imitant le site de liaison au récepteur d'un activateur du plasminogène.

10. Utilisation selon la revendication 1, dans laquelle ladite substance active est un mutant de l'activateur du plasminogène qui est résistant à l'endocytose à médiation par les récepteurs dans lesdites cellules mésothéliales.

11. Utilisation selon la revendication 1, dans laquelle ladite substance active est un vecteur de transfection ou d'infection recombinant pour lesdites cellules mésothéliales, lequel vecteur contient un acide nucléique codant pour un ARNm anti-sens pour un récepteur des cellules mésothéliales impliqué dans l'internalisation des activateurs du plasminogène et permet la modification génétique in vivo ou ex vivo desdites cellules mésothéliales de manière à réguler à la baisse leur expression dudit récepteur.

12. Utilisation selon la revendication 1, dans laquelle ladite substance active est capable de réguler à la baisse l'expression des récepteurs sur lesdites cellules mésothéliales, lesquels récepteurs sont impliqués dans l'internalisation des activateurs du plasminogène.
